# EUROPEAN PATENT APPLICATION

(11) **EP 1 652 531 A1**
(43) Date of publication of application: **03.05.2006**
(21) Application number: 05111027.8
(22) Date of filing: 22.09.1999
(51) Int. Cl.: A61K 38/26, A61P 9/10

(54) **Use of GLP-1 or Analogues in Treatment of Stroke**

(30) Priority: 24.09.1998 US 101719
(62) Divisional of application: 99951570.3
(71) Applicant: Eli Lilly & Company, Indianapolis, IN 46285 (US)
(72) Inventor: Saud, Effendic, S-18134 Lidingo (SE)
(74) Representative: Kent, Lindsey Ruth

(57) **Abstract**

This invention provides a method of reducing mortality and morbidity associated with stroke. GLP-1, a GLP-1 analogue, or a GLP-1 derivative is administered at a dose effective to normalize blood glucose.

## Description

This invention relates to methods and compositions for reducing mortality and morbidity after stroke by controlling hyperglycemia. The methods and compositions are particularly useful for non-insulin dependent diabetics, those at new risk of stroke or who have ongoing or recurring stroke. Pre-existing hyperglycemia is healed and new-onset hyperglycemia is prevented.

### BACKGROUND OF THE INVENTION

Morbidity and mortality from cardiovascular disease is higher in patients with manifest diabetes or impaired glucose tolerance compared to patients without those disorders. Diabetics account for up to 24% of the total number of patients admitted to coronary care units for suspected myocardial infarction, whereas they constitute only about 5% of the general population (Fuller, 1993). In-hospital mortality of diabetic patients with myocardial infarction is twice that of non-diabetics (Hamsten, 1994, Malmberg and Rydén, 1988). Diabetics experience more morbidity and die more often in the post-acute recovery phase, than at other periods; mostly due to fatal reinfarction and congestive heart failure (Stone, 1989, Karlson, 1993, Barbash, 1993). The difference in mortality and morbidity between diabetics and non-diabetics following stroke persists, despite reduction in the incidence of morbidity and mortality following acute myocardial infarction (Granger, 1993, Grines, 1993).

The risk of stroke is also known to be markedly elevated in patients with diabetes. Thus the risk of stroke in male patients with non-insulin-dependent diabetes (NIDDM) was about threefold and in NIDDM women fivefold higher than in corresponding nondiabetic subjects (Lehto, 1966). In another study in Finland, men with diabetes at baseline had sixfold increased risk of death from stroke whereas relative risk for men who developed diabetes during follow up was 1.7. In women the respective relative risks were 8.2 and 3.7 (Tuomilehto, 1996). Moreover another study has demonstrated that also mild and unrecognized hyperglycemia was a risk factor for acute stroke and that cumulative was raised in patients with an elevated blood glucose value irrespective of their HbA₁, values which reflect a long-term glycemic control (Gray, 1987).

The deteriorating effect of hyperglycemia on the outcome of stroke has been also demonstrated in other studies (Cazzato, 1991, Kiers, 1992, deFalco, 1993, Moulin, 1997, Weir, 1997). The intensity of stress hormone responses during stroke significantly contributes to development of hyperglycemia (O'Neill, 1992), but it is likely that hyperglycemia *per se* adversely affects ischemic brain metabolism mainly by prolonged acidosis (Levine, 1988, Wass and Lanier, 1996).

Studies in animals strongly support the idea that hyperglycemia significantly worsens brain damage during stroke due to reduction in regional cerebral blood flow, marked edema and brainstem compression, increased infarct size, increased systolic Ca²⁺ levels, lactate accumulation, bloodbrain barrier disruption and increased hemorrhage (Duckrow, 1985, 1987, de Courten-Myers, 1988, Slivka, 1991, Araki, 1992, Wagner, 1992, Dietrich, 1993, Broderick, 1995).

Palliative measures to normalize blood glucose and to control metabolic cascades that exacerbate stroke damage in diabetics are needed. This may be achieved by adjusted infusion of insulin and glucose, and post-acute tight regulation of blood glucose by subcutaneous multidose insulin treatment. The latter regime when used in treatment of diabetic patients during acute myocardial infarction lowered mortality during the year following myocardial infarction by 30% compared with a control group of diabetics who did not receive insulin treatment unless deemed necessary (Malmberg, 1995).

Insulin infusion, however, creates the potential for hypoglycemia, which is defined as blood glucose below 0.3 mM. Hypoglycemia increases the risk of myocardial infarction, ventricular arrhythmia and is a dangerous consequence of insulin infusion. An algorithm for insulin infusion for diabetics with stroke was developed to prevent hypoglycemia (Hendra, 1992). However, 21% of the patients developed hypoglycemia under this algorithm. In another study of glucose control following myocardial infarction, 18% of the patients developed hypoglycemia when infused with insulin and glucose (Malmberg, 1994).

Insulin infusion also requires frequent monitoring of blood glucose levels so that the onset of hypoglycemia can be detected and remedied as soon as possible. In patients receiving insulin infusion in the cited study (Malmberg, 1994), blood glucose was measured at least every second hour, and the rate of infusion was adjusted accordingly. Thus, the safety and efficacy of insulin-glucose infusion therapy depends on easy and rapid access to blood glucose data. Such an intense need for monitoring blood glucose places a heavy burden on health care professionals, and increases the inconvenience and cost of treatment. As a result, intensive care units often do not allocate resources for optimizing blood glucose levels in diabetics, as might be obtained by intravenous administration of insulin. Considering the risks and burdens inherent in insulin infusion, an alternate approach to management of blood glucose during acute stroke in diabetics is needed.

The incretin hormone, glucagon-like peptide 1, abbreviated as GLP-1, is processed from proglucagon in the gut and enhances nutrient-induced insulin release (Krcymann, 1987). Various truncated forms of GLP-1 are known to stimulate insulin secretion (insulinotropic action) and cAMP formation *(see, e.g.,* Mojsov, 1992). A relationship between various *in vitro* laboratory experiments and mammalian, especially human, insulinotropic responses to exogenous administration of GLP-1, GLP-1(7-36) amide, and GLP-1(7-37) acid has been established *(see, e.g.,* Nauck, 1993 a and b, Gutniak, 1992, and Thorens, 1993). GLP-1(7-36) amide exerts a pronounced antidiabetogenic effect in insulin-dependent diabetics by stimulating insulin sensitivity and by enhancing glucose-induced insulin release at physiological concentrations (Gutniak, 1992). When administered to non-insulin dependent diabetics, GLP-1(7-36) amide stimulates insulin release, lowers glucagon secretion, inhibits gastric emptying and enhances glucose utilization (Nauck, 1993 a and b, Gutniak, 1992).

The use of GLP-1 type molecules for prolonged therapy of diabetes has been obstructed because the serum half-life of such peptides is quite short. For example, GLP-1(7-37) has a serum half-life of only 3 to 5 minutes. GLP-1(7-36) amide has a half-life of about 50 minutes when administered subcutaneously. Thus, these GLP molecules must be administered as a continuous infusion to achieve a prolonged effect (Gutniak, 1994).

### BRIEF SUMMARY OF THE INVENTION

The present invention provides methods and compositions for reducing mortality and morbidity after stroke. The method includes administering a compound from the group consisting of GLP-1, GLP-1 analogs, GLP-1 derivatives, and pharmaceutically-acceptable salts thereof at a dose effective to normalize blood glucose, to a patient in need thereof The present invention provides the benefits of reduction in mortality and morbidity in diabetics after stroke, for example, by effecting smaller infarct size. The treatments in non-insulin dependant of the present invention compared to combined treatment with infusions of insulin and glucose avoids the inconvenient and expensive frequent monitoring of blood glucose as well as interpretation of blood glucose results and adjustment of insulin dose rate. The treatments also avoid the ever present risk ofhypoglycemia that accompanies insulin infusion. In the present invention, some GLP-1's have short half-lives and the consequent need for continuous administration are not disadvantages because the patient is typically bed ridden, in an intensive care unit, where fluids are continuously administered parenterally. This treatment includes all patients with hyperglycemia irrespective of whether they were diagnosed as diabetic.

### BRIEF DESCRIPTION OF THE DRAWINGS

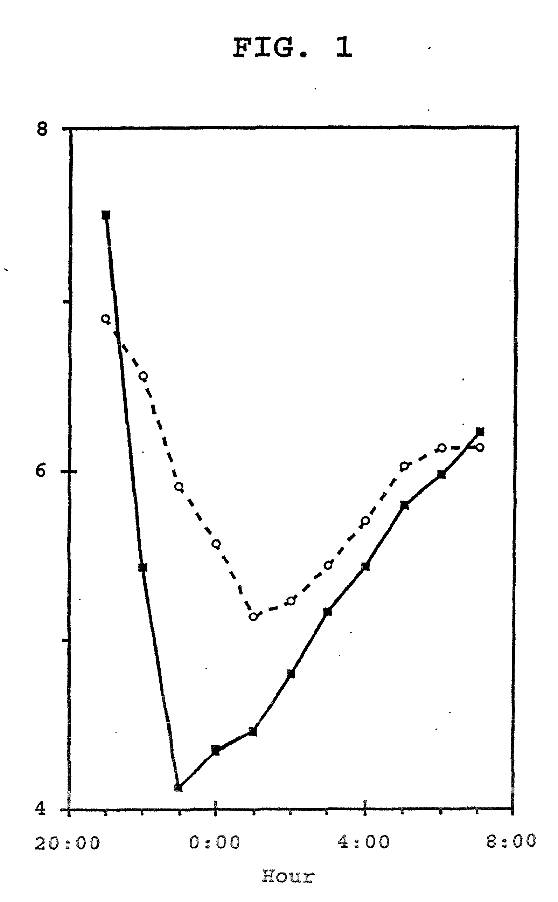

FIG. 1 is a graph showing the effect of continuous infusion GLP-1(7-36) amide on average blood glucose concentration (mM), (―■―) in five NIDDM patients during the night. The graph also depicts the effect of continuous insulin infusion on average blood glucose concentration (----O----) in the same five NIDDM patients, but on a different night.

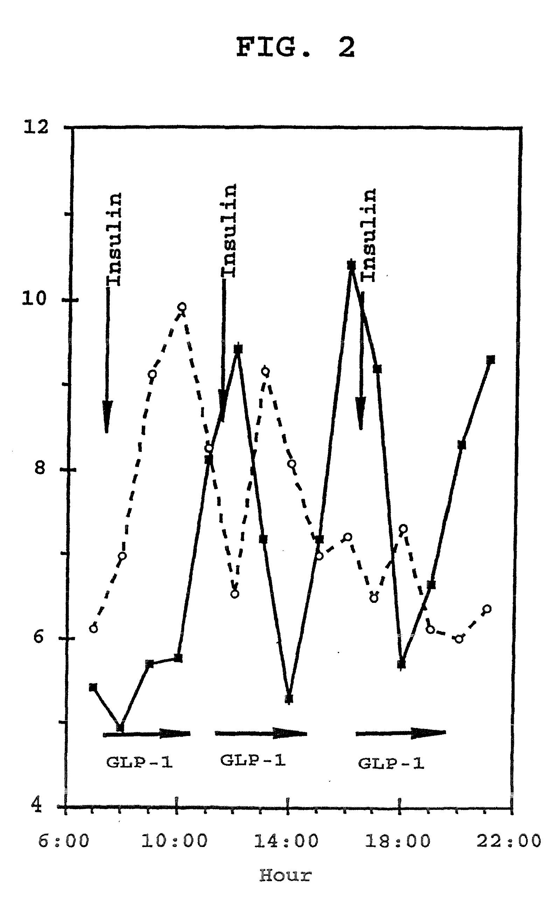

FIG. 2 is a graph showing the effect of GLP-1(7-36) amide infusion on average blood glucose concentration (mM) (―■―) in five NIDDM patients when infused during the day, for three hours starting at the beginning of each of three meals. The graph also depicts the effect of subcutaneous injection of insulin on average blood glucose concentration (----O----) in the same five NIDDM patients, but on a different day, and with injection shortly before each meal.

### DETAILED DESCRIPTION OF THE INVENTION

Methods and compositions, in particular medicaments (pharmaceutical compositions or formulations) using glucagon-like peptide-1, analogs or derivatives thereof, are effective in reducing mortality and morbidity after stroke in diabetic patients, in particular, in non-insulin dependent diabetics. Analogs and derivatives of GLP-1 that are useful for the practice of the invention are those with an increased half-life compared to GLP-1 and the ability to effect mortality and morbidity when administered to a subject.

### Stroke

Stroke or apoplexy or cerebrovascular accident (CVA) is a cerebrovascular disease characterized by an abrupt onset of a non-convulsive and focal neurological deficit. Stroke causes about 200,000 deaths in the United States each year as well as neurologic disability. In western countries ischemia-infarction cause stroke in about 85-90 percent of cases, whereas intracranial hemorrhages are found in the rest of the patient group. Cerebral ischemia is provoked by a reduction in blood flow lasting for several seconds. If the cessation of flow lasts for more than a few minutes, infarction of brain tissue evolves. The most common cause of cerebral ischemia and infarction are atherosclerosis with thromoboembolism and cardiogenic embolism. The ischemic stroke is characterized clinically by its mode of onset and subsequent course. The hallmark presentation is an acute onset of a hemiparesis in an individual in the atherosclerotic age group. However, any symptoms of brain dysfunction may occur. Symptoms and signs of carotid system disease often affect the distribution of the middle cerebral artery, and the patient may exhibit a contralateral hemiparesis, hemisensory deficit and hemianopsis. When the dominant hemisphere is involved, there is usually some degree of aphasia. The anterior (carotid) or posterior (vertebrobasilar) circulation may be also involved which results in more or less specific clinical symptoms.

Hyperglycemia is defined as a plasma glucose concentration of about 200 mg/dl (11.1 mmol/l) or greater, or a fasting plasma glucose level of about 125 mg/dl (7.0 mmol/l or greater). An aspect of the invention is to prevent hyperglycemia or to reduce it to normal values.

### Compounds

GLP-1 analogs, derivatives, variants, precursors and homologues are all suitable for the practice of the invention as long as the active fragment that effects reduced mortality or morbidity after stroke is included.

"GLP-1" means GLP-1(7-37). By custom in the art, the amino-terminus of GLP-1(7-37) has been assigned number 7 and the carboxy-terminus, number 37. The amino acid sequence of GLP-1(7-37) is well known in the art, but is presented below for the reader's convenience:

NH2-His7-Ala-Glu-Gly10-
Thr-Phe-Thr-Ser-Asp15-Val-Ser-Ser-Tyr-Leu20-
Glu-Gly-Gln-Ala-Ala25-Lys-Glu-Phe-Ile-Ala30-
Trp-Leu-Val-Lys-Gly35-Arg-Gly37-COOH

A "GLP-1 analog" is defined as a molecule having a modification including one or more amino acid substitutions, deletions, inversions, or additions when compared with-GLP-1. GLP-1 analogs known in the art include, for example, GLP-1(7-34) and GLP-1(7-35), GLP-1(7-36), Val⁸-GLP-1(7-37), Gln⁹-GLP-1(7-37), D-Gln⁹-GLP-1(7-37), Thr¹⁶-Lys¹⁸-GLP-1(7-37), and Lys¹⁸-GLP-1(7-37). Preferred GLP-1 analogs are GLP-1(7-34) and GLP-1(7-35), which are disclosed in U.S. Patent No. 5,118,666, and also GLP-1(7-36). These compounds are the biologically processed forms of GLP-1 having insulinotrapic properties. Other GLP-1 analogs are disclosed in U.S. Patent No. 5,545,618.

A "GLP-1 derivative" is defined as a molecule having the amino acid sequence of GLP-1 or of a GLP-1 analog, but additianally having at least one chemical madificatian of one or more of its amino acid side groups, α-carbon atoms, terminal amino group, or terminal carboxylic acid group. A chemical modification includes adding chemical moieties, creating new bonds, and removing chemical moieties. Modifications at amino acid side groups include acylation of lysine ∈-amino groups, N-alkylation of arginine, histidine, or lysine, alkylation of glutamic or aspartic carboxylic acid groups, and deamidation ofglutamine or asparagine. Modifications of the terminal amino include the des-amino, N-lower alkyl, N-di-lower alkyl, and N-acyl modifications. Modifications of the terminal carboxy group the amide, lower alkyl amide, dialkyl amide, and lower alkyl ester modifications. A lower alkyl is a C₁₋C₄ alkyl. Furthermore, one or more side groups, or terminal groups, may be protected by protective groups known to the ordinarily-skilled protein chemist. The α-carbon of an amino acid may be mono- or di-methylated.

In the present invention a preferred group of GLP-1 analogs and derivatives for use in the present invention is composed of the various GLP-1 molecules claimed in U.S. Patent No. 5,545,618. Effective analogs of the active GLP-1 peptides, 7-34, 7-35, 7-36 and 7-37 have amino acid substitutions at positions 7-10 and/or are truncated at the C-terminus and/or contain various other amino acid substitutions in the basic peptide. Analogs having D-amino acid substitutions in the 7 and 8 positions and/or N-alkylated or N-acylated amino acids in the 7 position are particularly resistant to degradation *in vivo.*

Analogs which show enhanced insulin stimulating properties have the sequence of native GLP-1, 7-34, 7-35, 7-36, or 7-37, or the C-terminal amide thereof, with at least one modification selected from the group consisting of:
(a) substitution of a neutral amino acid, arginine, or a D form of lysine for lysine at position 26 and/or 34 and/or a neutral amino acid, lysine, or a D form of arginine for arginine at position 36;
(b) substitution of an oxidation-resistant amino acid for tryptophan at position 31;
(c) substitution according to at least one of:
   Y for V at position 16;
   K for S at position 18;
   D for E at position 21;
   S for G at position 22;
   R for Q at position 23;
   R for A at position 24; and
   Q for K at position 26;
   (Using the single letter codes for amino acids)
(d) a substitution comprising at least one of:
   an alternative small neutral amino acid for A at position 8;
   an alternative acidic amino acid or neutral amino acid for E at position 9;
   an alternative neutral amino acid for G at position 10; and
   an alternative acidic amino acid for D at position 15; and
(e) substitution of an alternative neutral amino acid or the D or N-acylated or alkylated form of histidine for histidine at position 7.

With respect to modifications (a), (b), (d) and (e), the substituted amino acids may be in the D form. The amino acids substituted at position 7 can also be in the N-acylated or N-alkylated forms.

Peptides which show enhanced degradation resistance in plasma as compared to GLP-1(7-37) are suitable for the practice of the present invention. In these analogs, any of the above-mentioned truncated forms of GLP-1(7-34) or GLP-1(7-37) or their C-terminal amidated forms is modified by
(a) substitution of a D-neutral or D-acidic amino acid for H at position 7, or
(b) substitution of a D-amino acid for A at position 8, or
(c) both, or
(d) substitution of an N-acylated or N-alkylated form of any naturally occurring amino acid for H at position 7.

Thus analogs which are resistant to degradation include (N-acyl (1-6C) AA)⁷ GLP-1(7-37) and (N-alkyl (1-6C AA)⁷ GLP-1(7-37) wherein when AA is a lysyl residue, one or both nitrogens may be alkylated or acylated, AA symbolizes any amino acid consistent with retention of insulin stimulating activity.

For substitutions af D-amino acids in the 7 and 8 positions, the D residue of any acidic or neutral amino acid can be used at position 7 and of any amino acid at position 8, again consistent with insulin stimulating activity. Either or both of position 7 and 8 can be substituted by a D-amino acid; the D-amino acid at position 7 can also be acylated or alkylated. These modified forms are applicable not only to GLP-1(7-37) but also to shorter truncated analogs.

Thus, among the preferred analogs are those wherein the (7-34), (7-35), or (7-37) form of GLP-1 has been modified only by substitution of a neutral amino acid, arginine, or a D form of lysine for lysine at position 26 and/or 34 and/or a neutral amino acid, lysine, or a D form of arginine for arginine at position 36. Particularly preferred are those wherein the amino acid substituted for lysine at position 26 and 34 is selected from the group consisting of K⁺, G, S, A, L, I, Q, R, R⁺ and M, and for arginine at position 36 is selected from the group of K, K⁺, G, S, A, L, I, Q, M, and R⁺ (where ⁺ indicates a D form).

Also preferred are analogs wherein the sole modification is the substitution of an oxidation-resistant amino acid for tryptophan at position 31. Particularly favored substitutions are selected from the group consisting of F, V, L, I, A, and Y.

Particularly preferred are those analogs wherein combined substitutions of S for G at position 22, R at positions 23 and 24 for Q and A respectively, and Q for K at position 26 have been made, or substitutions of Y for V at position 16 and K for S at position 18 have been made, or these substitutions plus D for E at positions 21 have been made.

Particularly preferred among these analogs are those wherein the small neutral amino acid substituted for alanine at position 8 is selected from the group consisting of S, S⁺, GC, C⁺, Sar, A⁺, beta-ala and Aib; and/or the acidic or neutral amino acid substituted for glutamic acid at position 9 is selected from the group consisting of E⁺, D, D⁺, Cya, T, T⁺, N, N⁺, Q, Q⁺, Cit, MSO, and acetyl-K; and/or the alternative neutral amino acid substituted for glycine at position 10 is selected from the group consisting of S, S⁺, Y, Y⁺, T, T⁺, N, N⁺, Q, Q⁺, Cit, MSO, acetyl-K, F, and F⁺; and/or wherein D is substituted for E at position 15.

Also preferred are analogs wherein position 7 alone has been altered. Preferred substitutions are those wherein the amino acid substituted for histidine at position 7 is selected from the group consisting of H⁺, Y, Y⁺, F, F⁺, R, R⁺, Orn, Orn⁺, M, M⁺, N-formyl-H, N-formyl-H⁺, N-acetyl-H, N-acetyl-H⁺, N-isopropyl-H, N-isopropyl-H⁺, N-acetyl-K, N-acetyl-K⁺, P and P⁺.

Also preferred are embodiments with a combination of only two of the above-referenced classes of modified forms, in addition to the following specific embodiments (analogs):
(H⁺)⁷-GLP-1(7-37);
(Y)⁷-GLP-1(7-37);
(N-acetyl-H)⁷-GLP-1(7-37);
(N-isopropyl-H)⁷-GLP-1(7-37);
(A⁺)⁸-GLP-1(7-37);
(E⁺)⁹-GLP-1(7-37);
(D)⁹-GLP-1(7-37);
(D⁺)⁹-GLP-1(7-37);
(F⁺)¹⁰-GLP-1(7-37);
(S)²²(R)²³(R)²⁴(Q)²⁶-GLP-1(7-37);
(S)⁸(Q)⁹(Y)¹⁶(K)¹⁸(D)²¹-GLP-1(7-37).

Preferred forms of analogs with enhanced stability also have only one, or at most two, amino acid modifications.

Preferred substitutions for the histidine at position 7 include the D-forms of acidic or neutral amino acids of the D-forms of histidines. Preferred are P⁺, D⁺, E⁺, N⁺, Q⁺, L⁺, V⁺, I⁺ and H⁺.

The histidine at position 7, or a replacement (D or L), can also be N-alyklated (1-6C) or N-acylated (1-6C). Alkyl groups are straight or branched chain (including cyclic) hydrocarbyl residues of the indicated member of C. Acyl groups are of the formula RCO-wherein R is alkyl. Preferred alkyl groups are t-propyl, α-propyl and ethyl; preferred acyl are acetyl and propionyl. Preferred residues which may be alkylated or acylated include P, D, E, N, Q, V, L, I, K and H in either the D or L form.

Preferred substitutions for alanine at position 8 are the D-forms of P, V, L, I and A; also preferred are the D forms of D, E, N, Q, K, T, S and H.

Some specific analogs show both enhanced insulin release stimulating activity and enhanced stability.

A preferred group ofGLP-1 analogs and derivatives for use in the present invention is composed of molecules of the formula: 
R1-X-Glu-Gly10-
Thr-Phe-Thr-Ser-Asp15-Val-Ser-Ser-Tyr-Leu20-
Y-Gly-Gln-Ala-Ala25-Lys-Z-Phe-Ile-Ala30-
Trp-Leu-Val-Lys-Gly35-Arg-R2
 and the pharmaceutically-acceptable salts thereof, wherein: R₁ is selected from the group consisting of L-histidine, D-histidine, desamino-histidine, 2-amino-histidine, β-hydroxy-histidine, homohistidine, alpha-fluormethyl-histidine, and alpha-methyl-histidine; X is selected from the group consisting of Ala, Gly, Val, Thr, Ile, and alphamethyl-Ala; Y is selected the group consisting of Glu, Gln, Ala, Thr, Ser, and Gly; Z is selected from the group consisting of Glu, Gln, Ala, Thr, Ser, and Gly; and R₂ is selected from the group consisting of NH₂, and Gly-OH; provided that the compound has an isoelectric point in the rage from about 6.0 to about 9.0 and further providing that when R₁ is His, X is Ala, Y is Glu, and Z is Glu, R₂ must be NH₂.

Numerous GLP-1 analogs and derivatives having an isoelectric point in the range from about 6.0 to about 9.0 have been disclosed and include, for example:

GLP-1(7-36)NH2
Gly8-GLP-1(7-36)NH2
Gln9-GLP-1(7-37)
D-Gln9-GLP-1(7-37)
acetyl-Lys9-GLP-1(7-37)
Thr9-GLP-1(7-37)
D-Thr9-GLP-1(7-37)
Asn9-GLP-1(7-37)
D-Asn9-GLP-1(7-37)
Ser22-Arg23-Arg24-Gln26-GLP-1(7-37)
Thr16-Lys18-GLP-1(7-37)
Lys18-GLP-1(7-37)
Arg23-GLP-1(7-37)
Arg24-GLP-1(7-37)

Another preferred group of active compounds for use in the present invention is disclosed in WO 91/11457 (related to U.S. 5,545,618) and includes GLP-1(7-34), GLP-1(7-35), GLP-1(7-36), or GLP-1(7-37), or the amide form thereof, and pharmaceutically-acceptable salts thereof, having at least one modification including those shown below:
(a) substitution of glycine, serine, cysteine, threonine, asparagine, glutamine, tyrosine, alanine, valine, isoleucine, leucine, methionine, phenylalanine, arginine, or D-lysine for lysine at position 26 and/or position 34; or substitution of glycine, seine, cysteine, threonine, asparagine, glutamine, tyrosine, alanine, valine, isoleucine, leucine, methionine, phenylalanine, lysine, or a D-arginine for arginine at position 36;
(b) substitution of an oxidation-resistant amino acid for tryptophan at position 31;
(c) substitution of at least one of: tyrosine for valine at position 16; lysine for serine at position 18; aspartic acid for glutamic acid at position 21; serine for glycine at position 22; arginine for glutamine at position 23; arginine for alanine at position 24; and glutamine for lysine at position 26; and
(d) substitution of at least one of: glycine, serine, or cysteine for alanine at position 8; aspartic acid, glycine, serine, cysteine, threonine, asparagine, glutamine, tyrosine, alanine, valine, isoleucine, leucine, methionine, or phenylalanine for glutamic acid at position 9; serine, cysteine, threonine, asparagine, glutamine, tyrosine, alanine, valine, isoleucine, leucine, methionine, or phenylalanine for glycine at position 10; and glutamic acid for aspartic acid at position 15; and
(e) substitution of glycine, serine, cysteine, threonine, asparagine, glutamine, tyrosine, alanine, valine, isoleucine, leucine, methionine, or phenylalanine, or the D- or N-acylated or alkylated form of histidine for histidine at position 7; wherein, in the substitutions is (a), (b), (d), and (e), the substituted amino acids can optionally be in the D-form and the amino acids substituted at position 7 can optionally be in the N-acylated or N-alkylated form.

Because the enzyme, dipeptidyl-peptidase IV (DPP IV), may be responsible for the observed rapid *in vivo* inactivation of administered GLP-1 (Mentlein *et al.,* 1993), administration of GLP-1 analogs and derivatives that are protected from the activity of DPP IV is preferred, and the administration of Gly⁸-GLP-1(7-36)NH₂, Val⁸-GLP-1(7-37)OH, α-methyl-Ala⁸-GLP-1(7-36)NH₂, and Gly⁸-Gln²¹-GLP-1(7-37)OH Gly⁸-GLP-1(7-37)OH or pharmaceutically-acceptable salts thereof, is more preferred.

The use in the present invention of a molecule claimed in U.S. Patent No. 5,188,666 ('666) is also preferred. Such a molecule includes a peptide having one of the following amino acid sequences: 
NH2-His7-Ala-Glu-Gly10-
Thr-Phe-Thr-Ser-Asp15-Val-Ser-Ser-Tyr-Leu20-
Glu-Gly-Gln-Ala-Ala25-Lys-Glu-Phe-Ile-Ala30-
Trp-Leu-Val-X
 wherein X may be Lys and Lys-Gly; or a derivative of said peptide, and wherein said peptide may be a pharmaceutically-acceptable acid addition salt of said peptide; a pharmaceutically-acceptable carboxylate salt of said peptide; a pharmaceutically-acceptable lower alkylester of said peptide; or a pharmaceutically-acceptable amide of said peptide selected from the group consisting of amide, lower alkyl amide, and lower dialkyl amide.

The invention in '666 pertains to a peptide fragment which is insulinotropic and is derivable from a naturally occurring amino acid sequence. These fragments are suitable for the practice of the present invention. The invention comprises a compound selected from the group consisting of:
(A) a peptide comprising the sequence: 
His-Ala-Glu-Gly-Thr-Phe-Thr-Ser-Asp-Val-Ser-Ser-Tyr-Leu-Glu-Gly-
Gln-Ala-Ala-Lys-Glu-Phe-Ile-Ala-Trp-Leu-Val-X
 wherein X is selected from the group consisting af
   (a) Lys,
   (b) Lys-Gly,
   (c) Lys-Gly-Arg;
      and (B) a derivative of the peptide; wherein the compound is substantially free of natural contaminants, and has a insulinotropic activity which exceeds the insulinatropic activity of GLP-1 (1-36) or GLP-1 (1-37).

The invention also includes a compound selected from the group consisting of:
(A) a peptide comprising the sequence: 
His-Ala-Glu-Gly-Thr-Phe-Thr-Ser-Asp-Val-Ser-Ser-Tyr-Leu-Glu-Gly-
Gln-Ala-Ala-Lys-Glu-Phe-Ile-Ala-Trp-Leu-Val-X
 wherein X is selected from the group consisting of:
   (a) Lys,
   (b) Lys-Gly,
   (c) Lys-Gly-Arg;
      and (B) a derivative of the peptide; wherein the compound is substantially free of natural contaminants, and has an insulinotropic activity at a concentration of at least 10⁻¹⁰M.

Of particular interest are peptides of the following formula:
(1) 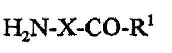 wherein R¹ is OH, OM, or -NR² R³;
   M is a pharmaceutically acceptable cation or a lower branched or unbranched alkyl group;
   R² and R³ are the same or different and selected from the group consisting of hydrogen and a lower branched or unbranched alkyl group;
   X is a peptide comprising the sequence:
      His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg
      NH₂ is the amine group of the amino terminus of X; and CO is the carbonyl group of the carboxy terminus of X;
(2) the acid addition salts thereof; and
(3) the protected or partially protected derivatives thereof;
   wherein said compound has an insulinotropic activity which exceeds the insulinotropic activity of GLP-1 (1-36) or GLP-1 (1-37).

Another preferred group of molecules for use in the present invention consists of compounds claimed in U.S. Patent No. 5,512,549 having the general formula: 
R¹-Ala-Glu-Gly¹⁰-
Thr-Phe-Thr-Ser-Asp¹⁵-Val-Ser-Ser-Tyr-Leu²⁰-
Glu-Gly-Gln-Ala-Ala²⁵-Xaa-Glu-Phe-Ile-Ala³⁰-
Trp-Leu-Val-Lys-Gly³⁵-Arg-R³
|
R²
 and pharmaceutically-acceptable salts thereof, wherein R¹ may be 4-imidazopropionyl, 4-imidazoacetyl, or 4-imidazo-α, α dimethyl-acetyl, R² may be C₆₋C₁₀ unbranched acyl, or absent; R³ may be Gly-OH or NH₂; and, Xaa is Lys or Arg.

More preferred compounds for use in the present invention are those in which Xaa is Arg and R² is a C₆-C₁₀ unbranched acyl.

Highly preferred compounds for use in the present invention are those in which Xaa is Arg, R² is C₆-C₁₀ unbranched acyl, and R³ is Gly-OH.

More highly preferred compounds for use in the present invention are those in which Xaa is Arg, R² is a C₆-C₁₀ unbranched acyl, R³ is Gly-OH, and R¹ is 4-imidazoproprionyl.

The most preferred compound for use in the present invention is that in which Xaa is Arg, R² is C₈ unbranched acyl, R³ is Gly-OH, and R¹ is 4-imidazoproprionyl.

The use in the present invention, a molecule claimed in U.S. Patent No. 5,120,712 is highly preferred. Such a molecule includes a peptide having the amino acid sequence: 
NH2-His7-Ala-Glu-Gly10-
Thr-Phe-Thr-Ser-Asp15-Val-Ser-Ser-Tyr-Leu20-
Glu-Gly-Gln-Ala-Ala25-Lys-Glu-Phe-Ile-Ala30-
Trp-Leu-Val-Lys-Gly35-Arg-Gly37-OH
 and a derivative of said peptide, wherein said peptide may be:
a pharmaceutically-acceptable acid addition salt of said peptide;
a pharmaceutically-acceptable carboxylate salt of said peptide;
a pharmaceutically-acceptable lower alkylester of said peptide; or
a pharmaceutically-acceptable amide of said peptide, wherein the amide may be an amide, lower alkyl amide, or lower dialkyl amide.

The use of GLP-1(7-36) amide, or a pharmaceutically-acceptable salt thereof, in the present invention is most highly preferred. The amino acid sequence of GLP-1(7-36) amide is:

NH2-His7-Ala-Glu-Gly10-
Thr-Phe-Thr-Ser-Asp15-Val-Ser-Ser-Tyr-Leu20-
Glu-Gly-Gln-Ala-Ala25-Lys-Glu-Phe-Ile-Ala30-
Trp-Leu-Val-Lys-Gly35-Arg-NH2

The use of Val⁸-GLP-1(7-37)OH, or a pharmaceutically-acceptable salt thereof, in the present invention is most highly preferred. The amino acid sequence of Val⁸-GLP-1(7-37)OH is:

NH2-His7-Ala-Glu-Gly10-
Thr-Phe-Thr-Ser-Asp15-Val-Ser-Ser-Tyr-Leu20-
Glu-Gly-Gln-Ala-Ala25-Lys-Glu-Phe-Ile-Ala30-
Trp-Leu-Val-Lys-Gly35-Arg-Gly37-OH

### Preparation of the Compounds

Methods for preparing the active compounds used in the present invention, namely, GLP-1, an GLP-1 analog, or a GLP-1 derivative, or any related compound including an active fragment effective in reduction of mortality or morbidity after stroke when administered peripherally, are well-known, and are described in U.S. Patent Nos. 5,118,666; 5,120,712; and 5,523,549.

The amino acid portion of the active compound used in the present invention, or a precursor thereto, is made by 1) solid-phase synthetic chemistry; 2) purification of GLP molecules from natural sources; 3) recombinant DNA technology; or 4) a combination of these methods.

Solid phase chemical synthesis of polypeptides is well known in the art and may be found in general texts in the area such as Dugas and Penney (1981); Merrifield (1962); Stewart and Young (1969, 1984).

For example, the amino acid portion may be synthesized by solid-phase methodology utilizing a 430A peptide synthesizer (PE-Applied Biosystems, Inc., 850 Lincoln Center Drive, Foster City, CA 94404) and synthesis cycles supplied by PE-Applied Biosystems. BOC-amino acids and other reagents are commercially available from PE-Applied Biosystems and other chemical supply houses. Sequential BOC chemistry using double couple protocols are applied to the starting p-methyl benzhydryl amine resins for the production of C-terminal carboxamides. For the production of C-terminal carboxamides. For the production of C-terminal acids, the corresponding PAM resin is used. Asn, Gln, and Arg are coupled using preformed hydroxy benzotriazole esters. The following side chain protecting groups may be used:
Arg, Tosyl
Asp, cydohexyl
Crlu, cyclohexyl
Ser, Benzyl
Thr, Benzyl
Tyr, 4-bromo carbobenzoxy

BOC deprotection may be accomplished with trifluoroacetic acid in methylene chloride. Following completion of the the peptides may be deprotected and cleaved from the resin with anhydrous hydrogen fluoride (HF) containing 10% meta-cresol. Cleavage of the side chain protecting group(s) and of the peptide from the resin is carried out at -5°C to 5°C, preferably on ice for 60 minutes. After removal of the HF, the peptide/resin is washed with ether, and the peptide extracted with glacial acetic acid and lyophilized.

Techniques well-known to the ordinarily-skilled artisan in recombinant DNA technology may be used to prepare the active compound used in present invention. In fact, recombinant DNA methods may be preferable because of higher yield. The basic steps in recombinant production are:
a) isolating a natural DNA sequence encoding a GLP-1 molecule of the present invention or constructing a synthetic or semi-synthetic DNA coding sequence for a GLP-1 molecule,
b) placing the coding sequence into an expression vector in a manner suitable for expressing proteins either alone or as a fusion proteins,
c) transforming an appropriate eukaryotic or prokaryotic host cell with the expression vector,
d) culturing the transformed host cell under conditions that will permit expression of a GLP-1 molecule, and
e) recovering and purifying the recombinantly produced GLP-1 molecule.

As previously stated, the coding sequences may be wholly synthetic or the result of modifications to the larger native glucagon-encoding DNA. A DNA sequence that encodes preproglucagon is presented in Lund *et al.* (1982) and may be used as starting material in the semisynthetic production of the compounds of the present invention by altering the native sequence to achieve the desired results.

Synthetic genes, the *in vitro* or *in vivo* transcription and translation of which results in the production of a GLP-1 molecule, may be constructed by techniques well known in the art. Owing to the natural degeneracy of the genetic code, the skilled artisan will recognize that a sizable yet definite number of DNA sequences may be constructed, all of which encode GLP-1 molecules of the present invention.

The methodology of synthetic gene construction is well-known in the art (Brown *et al*., 1979). The DNA sequence is designed from the desired amino acid sequence using the genetic code, which is easily ascertained by the ordinarily-skilled biologist. Once designed, the sequence itself may be generated using conventional DNA synthesizing apparatus such as the Model 380A or 380B DNA synthesizers (PE-Applied Biosystems, Inc., 850 Lincoln Center Drive, Foster City, CA 94404).

To express the amino acid portion of a compound used in the present invention, an engineered synthetic DNA sequence is inserted in any one of many appropriate recombinant DNA expression vectors through the use of appropriate restriction endonucleases (Maniatis *et al.,* 1989). Restriction endonuclease cleavage sites are engineered into either end of the GLP-1 molecule encoding DNA to facilitate isolation from, and integration into, amplification and expression vectors well-known in the art. The particular endonucleases employed will be dictated by the restriction endonuclease cleavage pattern of the parent expression vector employed. Restriction sites are chosen to properly orient the coding sequence with control sequences, thereby achieving proper in-frame reading and expression of the protein of interest. The coding sequence must be positioned to be in proper reading frame with the promoter and ribosome binding site of the expression vector, both of which are functional in the host cell in which the protein is to be expressed.

To achieve efficient transcription of the synthetic gene, it must be operably associated with a promoter-operator region. Therefore, the promoter-operator region of the synthetic gene is placed in the same sequential orientation with respect to the ATG start codon of the synthetic gene.

A variety of expression vectors useful for transforming prokaryotic and eukaryotic cells are well known in the art (Promega Catalogue, 1992, Stratagene Catalogue, 1992). Also, U.S. Patent No. 4,710,473 describes circular DNA plasmid transformation vectors useful for expression of exogenous genes in *E. coli* at high levels. These plasmids are useful as transformation vectors in recombinant DNA procedures and
(a) confer on the plasmid the capacity for autonomous replication in a host cell;
(b) confer autonomous plasmid replication in relation to the temperature at which host cell cultures are maintained;
(c) stabilize maintenance of the plasmid in host cell populations;
(d) direct synthesis of a protein product indicative af plasmid maintenance in a host cell population;
(e) provide in-series restriction endonuclease recognition sites unique to the plasmid; and
(f) terminate mRNA transcription.

These circular DNA plasmids are useful as vectors in recombinant DNA procedures for securing high levels of expression of exogenous genes.

Having constructed an expression vector for the amino acid portion of a compound used in the present invention, the next step is to place the vector into a suitable cell and thereby construct a recombinant host cell useful for expressing the polypeptide. Techniques for transforming cells with recombinant DNA vectors are well known in the art and may be found in such general references as Maniatis, *et al. supra.* Host cells may be constructed from either eukaryotic or prokaryotic cells.

Prokaryotic host cells generally produce the protein at higher rates and are easier to culture. Proteins expressed in high-level bacterial expression systems characteristically aggregate in granules or inclusion bodies, which contain high levels of the overexpressed protein. Such protein aggregates typically must be recovered, solubilized, denatured and refolded using techniques well known in the art (Kreuger *et al.,* 1990; U.S. Patent No. 4,923,967).

### Preparation of GLP-1 Analogs and Derivatives

Alterations to a precursor GLP-1 or GLP-1 amino acid sequence to produce a desired GLP-1 analog or GLP-1 derivative, or active fragment thereof, are made by well-known methods: chemical modification, enzymatic modification, or a combination of chemical and enzymatic modifications. The techniques of classical solution phase methods and semi-synthetic methods may also be useful for preparing the GLP-1 molecules used in the present invention. Methods for preparing the GLP-1 molecules of the present invention are well known to an ordinarily skilled peptide chemist.

Addition of an acyl group to the epsilon amino group of Lys³⁴ may be accomplished using any one of a variety of methods known in the art *(Bioconjugate Chem.* 1990, Hashimoto *et al.,* 1989).

For example, an N-hydroxy-succinimide ester of octanoic acid can be added to the lysyl-epsilon amine using 50% acetonitrile in borate buffer. The peptide can be acylated either before or after the imidazolic group is added. Moreover, if the peptide is prepared recombinantly, acylation prior to enzymatic cleavage is possible. Also, the lysine in the GLP-1 derivative can be acylated as taught in WO 96/29342.

The existence and preparation of a multitude of protected, unprotected, and partially-protected, natural and unnatural, functional analogs and derivatives of GLP-1 (7-36) amide and GLP-1 (7-37) molecules have been described (U.S. Pat. Nos. 5,120,712; 5,545,618 and 5,118,666; Orskov *et al.,* 1989; WO 91/11457).

Optionally, the amino and carboxyl terminal amino acid residues of GLP-1 derivatives may be protected, or, optionally, only one of the termini is protected. Reactions for the formation and removal of such protecting groups are described in works known to those of skill in the art including, for example, *Protective Groups in Organic Chemistry* 1973, Green, 1981, and Schröder and Lübke, 1965. Representative amino-protecting groups include, for example, formyl, acetyl, isopropyl, butoxycarbonyl, fluorenylmethoxycarbonyl, carbobenzyloxy, and the like. Representative carboxy-protecting groups include, for example, benzyl ester, methyl ester, ethyl ester, *t*-butyl ester, *ρ*-nitro phenyl ester, and the like.

Carboxyl-terminal, lower-alkyl-ester, GLP-1 derivatives used in the present invention are prepared by reacting the desired (C₁-C₄) alkanol with the desired polypeptide in the presence of a catalytic acid such as hydrochloric acid. Appropriate conditions for such alkyl ester formation include a reaction temperature of about 50°C and reaction time of about 1 hour to about 3 hours. Similarly, alkyl ester derivatives of the Asp and/or Glu residues can be formed.

Preparation of a carboxamide derivative of a compound used in the present invention is formed, for example, as described in Stewart *et al.* (1984).

A pharmaceutically-acceptable salt form of GLP-1, of a GLP-1 analog, or of a GLP-1 derivative may be used in the present invention. Acids commonly employed to form acid addition salts are inorganic acids such as hydrochloric acid, hydrobromic acid, hydriodic acid, sulfuric acid, phosphoric acid, the like, and organic acids such as *p*-toluenesulfonic acid, methanesulfonic acid, oxalic acid, p-bromophenyl-sulfonic acid, carbonic acid, succinic acid, citric acid, benzoic acid, acetic acid, and the like. Examples of such salts include the sulfate, pryrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydrozybenzoate, methoxybenzoate, phthalate, sulfonate, xylenesulfonate, phenylacetate, phenylpropionate, phenylbutyrate, citrate, lactate, gamma-hydroxybutyrate, glycolate, tartrate, methanesulfonate, propanefulfonate, naphthalene-1-sulfonate, napthalene-2-sulfonate, mandelate, and the like. Preferred acid addition salts are those formed with mineral acids such as hydrochloric acid and hydrobromic acid.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, and the like. The salt forms are particularly preferred.

A GLP-1, GLP-1 analog, or GLP-1 derivative used in the present invention may be formulated with one or more excipients before use in the present invention. For example, the active compound used in the present invention may be complexed with a divalent metal cation by well-known methods. Such metal cations include, for example, Zn^{++,} Mn⁺⁺, Fe⁺⁺, Co⁺⁺, Cd⁺⁺, Ni⁺⁺, and the like.

### Compositions of the Invention

Optionally, the active compound used in the present invention may be combined with a pharmaceutically-acceptable buffer, and the pH adjusted to provide acceptable stability, and a pH acceptable for parenteral administration.

Optionally, one or more pharmaceutically-acceptable anti-microbial agents may be added. Meta-cresol and phenol are preferred pharmaceutically-acceptable anti-microbial agents. One or more pharmaceutically-acceptable salts may be added to adjust the ionic strength or tonicity. One or more excipients may be added to further adjust the isotonicity of the formulation. Glycerin is an example of an isotonicity-adjusting excipients.

GLP-1 receptors and the signal transduction cascade initiated by ligand binding to the GLP-1 receptor are described in WO 96/25487; Thorens *et al.,* 1993; and Widmann *et al.,* 1994. The GLP-1 receptor is a membrane protein with seven transmembrane domains, coupled to heterotrimeric G-proteins that link activation of the receptor by ligand binding to production of intracellular secondary messengers, especially, cyclic adenosine monophosphate (cAMP). cAMP, in turn, activates a specific protein kinase, cAMP-dependent protein kinase (protein kinase A, PKA). This enzyme phosphorylates a number of key response elements present in the promoter region of certain genes. In pancreatic β-cells and other neuroendocrine cells, phosphorylation of some specific proteins of the regulated secretary pathway stimulates peptide secretion by stimulating exocytosis of secretory granules.

Various compounds are known to stimulate secretion of endogenous GLP-1. For example, exposure of STC-1 cells to certain secretagogues, such as, the adenylate cyclase activator, forskolin, or the protein kinase-C-stimulating agent, 12-O-tetradecanoylphobol-13-acetate (TPA), caused significant increases in release af GLP-1 (Abella *et al.,* 1994). The STC-1 cell line originated from an intestinal tumor in transgenic mice carrying insulin-promoting oncogenes, and STC-1 cells are known to contain mRNA transcripts of pro-glucagon, from which GLP-1 is generated. Other compounds, such as, somatostatin, gastric inhibitor polypeptide, glucose-dependent insulinotropic peptide, bombesin, calcitonin gene-related peptide, gastrin-releasing peptide, cholinergic agonists, the β-adrenergic agonist, isoproterenol, and the muscarinic cholinergic agonist, bethanechol, are similarly known to cause release of endogenous GLP-1 (Plarsancie *et al.,* 1994, Orskov *et al.,* 1986, Brubaker, 1991, Buchon, *et al.,* 1987).

### Administration of Compositions

Administration may be via any route known to be effective by the physician of ordinary skill, except that parenteral administration directly into the central nervous system is note a route taught or claimed in this invention. Peripheral, parenteral administration is preferred. Parenteral administration is commonly understood in the medical literature the injection of a dosage form the body by a sterile syringe or some other mechanical device such as an infusion pump. For the purpose of this invention, peripheral parenteral routes include intravenous, intramuscular, subcutaneous, and intraperitoneal routes of administration. Intravenous, intramuscular, and subcutaneous routes of administration of the compounds used in the present invention are more preferred. Intraveous and subcutaneous routes of administration of the compounds used in the present invention are yet more highly preferred. For parenteral administration, an active compound used in the present invention preferably is combined with distilled water at an appropriate pH.

Certain compounds used in the present invention to effect reduction in mortality and morbidity may also be amenable to administration by the oral, rectal, nasal, or lower respiratory routes, which are non-parenteral routes. Of the said non-parenteral routes, the lower respiratory route is preferred for administration of peptides used in the instant invention. Various formulations of peptide compounds for administration by the lower respiratory tract are disclosed in U.S. Patent Nos. 5,284,656 and 5,364,838. Publication WO 96/19197 discloses aerosol formulations of various peptides suitable for enhancing lower respiratory tract absorption of the compounds used in the instant invention. The oral route of administration is preferred for compounds used in the instant invention.

Additional pharmaceutical methods may be employed to control the duration of action. Controlled release preparations may be achieved by the use of polymers to complex or absorb the active compound used in the present invention. Extended duration may be obtained by selecting appropriate macromolecules, for example, polyesters, polyamino acids, polyvinylpyrrolidone, ethylenevinyl acetate, methylcellulose, carboxymethylcellulose, or pratamine sulfate, and by selecting the concentration of macromolecules, as well as the methods of incorporation, in order to prolong release. Another possible method to extend the duration of action by controlled release preparations is to incorporate an active compound used in the present invention into particles of a polymeric material such as polyesters,polyamino acids, hydrogels, poly (lactic acid) or ethylene vinylacetate copolymers. Alternatively, instead of incorporating a compound into these polymeric particles, it is possible to entrap a compound used in the present invention in microcapsules prepared, for example, by coacervation techniques or by interfacial polymerization, for example, hydroxymethylcellulose or gelatin-microcapsules, respectively, or in colloidal drug delivery systems, for example, lipsomes, albumin microspheres, microemulsions, nanoparticles, and nanocapsules, or in macroemulsions. Such teachings are known to those of skill in the art and disclosed, e.g. in *Remington's Pharmaceutical Sciences,* 1980.

### Dose

The dose of GLP-1, GLP-1 analog, or GLP-1 derivatives, or active fragments effective in a particular subject to reduce mortality and morbidity due to stroke will depend on a number of factors, among which are included the subject's sex, weight and age, stroke severity, stroke subtype, the route of administration and bioavailability, the persistence of the administered compound in the body, the formulation, and the potency. Where administration is intermittent, the dose per administration should also take into account the interval between doses, and the bioavailability of the administered compound. Where administration is continuous, a suitable dosage rate is between 0.25 and 6 pmol/kg/min. preferably from about 0.5 to about 1.2 pmol/kg/min. It is within the skill of the ordinary physician to titrate the dose and rate of administration of compositions containing GLP-1, GLP-1 analogs, or GLP-1 derivatives, or active fragments thereof to achieve the desired clinical result, reduction of mortality and morbidity after stroke by control of glucose. In an embodiment, reducing the plasma glucose concentration below about 7 mmol/l is a goal after acute stroke.

"Pharmaceutically acceptable" means suitable for administration to a human, that is, does not contain toxic elements, undesirable contaminants or the like, and does not interfere with the activity of the active compounds therein.

Numerous GLP-1 analogs and derivatives having an isoelectric point in the range 4.8 to 7.5 have been disclosed and include, for example:

GLP-1(7-36)NH2
Gly8-GLP-1(7-36)NH2
Gln9-GLP-1(7-37)

### Diagnosis of Stroke

A diagnosis of "stroke" is one involving medical judgment. The treatment which is the subject of this invention is generally given to a person during the acute phase of stroke.

A patient in need of the compounds used in the present invention is one who is in the acute phase of stroke, and who also is incapable of auto-regulation of blood glucose. A patient is incapable of auto-regulation if that patient: (1) was previously diagnosed with insulin-dependent diabetes (IDDM) or non-insulin dependent diabetes (NIDDM), according to the definitions of the National Diabetes Data Group *(Diabetes,* 1979); (2) has a blood glucose level greater than 11 mmol/liter, even without a previous diagnosis of diabetes; or (3) has an abnormal glucose tolerance.

The dose of GLP-1, GLP-1 analog, or GLP-1 derivative effective to normalize a patient's blood glucose level will depend on a number of factors, among which are included, without limitation, the patient's sex, weight and age, the severity of inability to regulate blood glucose, the underlying causes of inability to regulate blood glucose, whether glucose, or another carbohydrate source is simultaneously administered, the route of administration and bioavailability, the persistence in the body, the formulation, and the potency. Where administration is continuous, a suitable dosage rate is between 0.25 and 6 pmol/kg body weight/min, preferably from about 0.5 to about 1.2 pmol/kg/min. Where administration is intermittent, the dose per administration should take into account the interval between doses, the bioavailability of the GLP-1, GLP-1 analog, or GLP-1 derivative, and the level needed to effect normal blood glucose. It is within the skill of the ordinary physician to titrate the dose and rate of administration of GLP-1 and GLP-1 analog, or GLP-1 derivative to achieve the desired clinical result.

### EXAMPLES

The present invention will be more readily understood by reference to specific examples, which are provided to illustrate embodiments of the present invention.

### Example 1: Effects of Subcutaneous Infusion of GLP-1 (7-30) on Blood Glucose in Persons with NIDDM

GLP-1(7-36) amide was administered by a subcutaneous infusion at a dose rate of 1.2 pmol/kg/hr, for ten hours during the night, to five patients having non-insulin dependent diabetes (NIDDM). As a control, insulin was continuously infused in the same five patients, but on a different day than the GLP-1(7-36) amide infusion. The rate of insulin infusion was adjusted every two hours to achieve optimum control, and to avoid hypoglycemia. As demonstrated by the data in Table 1, and in FIG. 1, subcutaneous infusion of GLP-1(7-36) amide nearly normalized blood glucose without inducing hypoglycemia in any of the patients. The metabolic control with GLP-1(7-36) amide was better than that achieved by insulin, and the average blood glucose level was lower for GLP-1(7-36) amide treatment than for the control by a statistically significant amount at 23:00, 0:00, and at 1:00.

**Table 1. Average blood glucose levels for five NIDDM patients continuously infused for ten hours during the night with GLP-1(7-36) amide. In a control study with the same patients on a different day, insulin was administered by a continuous infusion.**

| | GLP-1 Infusion | | Insulin Infusion (Control) | |
|---|---|---|---|---|
| Hour | Average Blood Glucose (mM) | Std. Error (mM) | Average Blood Glucose (mM) | Std. Error (mM) |
| 21:00 | 7.5 | 0.45 | 6.9 | 0.68 |
| 22:00 | 5.4 | 0.76 | 6.6 | 0.5 5 |
| 23:00 | 4.1 | 0.16 | 5.9 | 0.98 |
| 0:00 | 4.4 | 0.23 | 5.6 | 0.90 |
| 1:00 | 4.4 | 0.29 | 5.1 | 0.58 |
| 2:00 | 4.8 | 0.34 | 5.2 | 0,58 |
| 3:00 | 5.2 | 0.41 | 5.4 | 0.30 |
| 4:00 | 5.4 | 0.41 | 5.7 | 0.25 |
| 5:00 | 5.8 | 0.41 | 6.0 | 0.30 |
| 6:00 | 6.0 | 0.45 | 6.1 | 0.38 |
| 7:00 | 6.2 | 0.45 | 6.1 | 0.33 |

### Example 2: Effects of Subcutaneous Infusion of GLP-1 (7-36) During Meals on Blood Glucose Levels of Persons with NIDDM

During the day, GLP-1(7-36) amide was infused into five NIDDM patients for three hours during breakfast, lunch, and dinner. The infusion times were 7:30-10:30 (breakfast), 10:30-1:30 (lunch), and 4:30-7:30 (dinner), as indicated in FIG. 2. In a control experiment in the same five NIDDM patients conducted on a different day, insulin was injected subcutaneously just before the start of the meals, as indicated in FIG. 2. While GLP-1 was infused, the post-prandial glucose excursions observed with insulin injection were eliminated, and normal blood glucose levels were maintained. Immediately after terminating each GLP-1(7-36) amide infusion, the blood glucose level increased significantly. No untoward side effects of GLP-1(7-3 6) amide were observed. These data indicate that GLP-1(7-36) amide infusion more effectively controls post-prandial glucose levels than insulin injection, and that the control is effective as long as GLP-1(7-36) amide infusion is continued.

**Table 2. Average blood glucose levels for five NIDDM patients infused with GLP-1(7-36) amide for three hours, beginning at the start of each meal. In a control study with the same patients on a different day, insulin was administered by subcutaneous injection just before each meal. Meals began at 7:30, 10:30, and at 16:30.**

| | GLP-1 Infusion | | Insulin Subcutaneous Injection | |
|---|---|---|---|---|
| Hour | Average Blood Glucose (mM) | Std. Error (mM) | Average Blood Glucose (mM) | Std. Error (mM) |
| 7:00 | 5.4 | 0.35 | 6.1 | 0.41 |
| 8:00 | 4.9 | 0.38 | 7.0 | 0.51 |
| 9:00 | 5.7 | 0.59 | 9.1 | 0.74 |
| 10:00 | 5.8 | 1.06 | 9.9 | 0.78 |
| 11:00 | 8.1 | 0.94 | 8.2 | 0.76 |
| 12:00 | 9.4 | 0.59 | 6.5 | 0.74 |
| 13:00 | 7.2 | 1.18 | 9.1 | 0.90 |
| 14:00 | 5.3 | 1.21 | 8.1 | 0.91 |
| 15:00 | 7.2 | 0.71 | 7.0 | 0.87 |
| 16:00 | 10.4 | 0.26 | 7.2 | 0.57 |
| 17:00 | 9.2 | 1.06 | 6.5 | 0.59 |
| 18:00 | 5.7 | 1.59 | 7.3 | 0.65 |
| 19:00 | 6.6 | 0.94 | 6.1 | 0.59 |
| 20:00 | 8.3 | 0.71 | 6.0 | 0.41 |
| 21:00 | 9.3 | 0.71 | 6.4 | 0.44 |

### DOCUMENTS CITED

Abello, Jr. *et al., Endocrinol.* 134:2011-2017a (1994).
Araki, N., *et al., Journal of Cerebral Blood Flow and Metabolism* 12:469-476 (1992).
Barbash, G.I., *et al., J. Am. Coll. Cardiol.* 22:707-713 (1993).
*Bioconjugate Chem.* "Chemical Modifications of Proteins: History and Applications," pp. 1-212 (1990).
Broderick, J.P., *et al., Stroke* 26:484-487 (1995).
Brown, *et al.* (1979) *Methods in Enzymology,* Academic Press, N.Y., Vol. 68, pp. 109- 151.
Brubaker, P.L., *Endocrinol.* 128;3175-03182 (1991).
Buchan, A.M.J., *et al., Gastroenterol.* 93:791-800 (1987).
Cazzato, G., *et al., Italian Journal of Neurological Sciences* 12:283-288 (1991).
deCourten-Myers, G., *et al., Stroke* 19:623-630 (1988).
deFalco, F.A., *et al., Schweizer Archiv Für Neurologie und Psychatrie* 144:233-239 (1993).
National Diabetes Data Group, *Diabetes* 28:1039-1057 (1979).
Dietrich, W.D*., et al., Stroke* 24:111-116 (1993).
Duckrow, R.B., *et al., Annals of Neurology* 17:262-272 (1985).
Duckrow, R.B., *et al., Stroke* 18:52-58 (1987).
Dugas, H. and Penney, C., *Bioorganic Chemistry,* Springer-Verlag, New York (1981), pp. 54-92.
Fuller, J.H., *Diabet. Metab.* 19:96-99 (1993).
Granger, C.B., *et al., J. Am. Coll. Cardiol.* 21(4):920-925 (1993).
Gray, C.S., *et al., Diabetic Medicine* 4:237-240 (1987).
Green, T.H., *Protective Groups in Organic Synthesis,* Wiley, New York (1981).
Grines, C., *et al., N. Engl. J. Med.* 328:673-679 (1993).
Gutniak, M., *et al., Diabetes Care* 17:1039-1044 (1994).
Gutniak, M., *et al., New England J: of Medicine* 326(20):1316-1322 (1992).
Hamsten, A., *et al., J. Int. Med.* 736:1-3 (1994) 236 Suppl.
Hashimoto *et al., Pharmaceutical Res.* 6(2):171-176 (1989).
Hendra, T.J., *et al., Diabetes Res. Clin. Pract.* 16:213-220 (1992).
Karlson, B.W., *et al., Diabet. Med.* 10(5):449-454 (1993).
Kiers, L., *et al., Journal of Neurology, Neurosurgery, Psychiatry* 55:263-270 (1992).
Krcymann, B., *et al., Lancet* 2:1300-1303 (1987).
Kreuger, *et al.* (1990) in *Protein Folding,* Gierasch and King, eds. pp. 13 6-142,
American Association for the Advancement of Science Publication No. 89-18S, Washington, D.C.
Lehto, S., *et al., Stroke* 27:63-8 (1966).
Levine, S.R., *Annals of Neurology* 23:416-418 (1988).
Lund, *et al., Proc. Natl. Acad. Sci. U.S.A.* 79:345-349 (1982).
Malmberg, K. and Rydén, L., *Eur. Heart J.* 9:256-264 (1988).
Malmberg, K., *et al., J. Am. College Cardiology* 26:57-65 (1995).
Malmberg, K.A., *et al., Diabetes Care* 17:1007-1014 (1994).
Maniatis *et al., Molecular Cloning: A Laboratory Manual,* Cold Springs Harbor Laboratory Press, N.Y., Vol. 1-3 (1989).
Mentlein, R., *et al., Eur. J. Biochem.* 214:829-835 (1993).
Merrifield, J.M., *Chem. Soc.* 85:2149 (1962).
Mojsov, S., *Int. J. Peptide Protein Research* 40:333-343 (1992).
Moulin, T., *et al., European Neurology* 38:10-27 (1997).
Nauck, M.A., *et al*., *Diabetologia* 36:741-744.
Nauck, M.A., *et al., J. Clin. Invest.* 91:301-307 (1993).
O'Neill, P.A., *et al., Stroke* 22:842-847 (1992).
Orskov, C., *et al., Endocrinol.* 119:1467-1475 (1986).
Orskov, C., *et al., J. Biol, Chem.* 264(22):12826-12829 (1989).
Plaisancie, P., *et al., Endocrinol.* 135:2348-2403 (1994).
*Protective Groups in Organic Chemistry,* Plenum Press, London and New York (1973).
*Remington's Pharmaceutical Sciences* (1980).
Schröder and Lübke, *The Peptides,* Vol. 1, Academic Press, London and New York (1965).
Silvka, A.P., *Brain Research* 562:66-70 (1991).
Stewart and Young, *Solid Phase Peptide Synthesis,* Freeman, San Francisco (1969), pp. 24-66.
Stewart, J.M., *et al., Solid Phase Peptide Synthesis,* Pierce Chemical Company Press (1984).
Stone, P., *et al., J. Am. Coll. Cardiol.* 14:49-57 (1989).
*The Promega Biological Research Products Catalogue* (1992) (Promega Corp., 2800 Woods Hollow Road, Madison, WI 53711-5399)
*The Stratagene Cloning Systems Catalogue* (1992) (Stratagene Corp., 11011 North
Torrey Pines Road, La Jolla, CA 92037)
Thorens, B., *et al., Diabetes* 42:1219-1225 (1993).
Tuomilehto, J., *et al., Stroke* 27:210-215 (1996).
Wagner, K.R, *et al., Journal of Cerebral Blood Flow and Metabolism* 12:213-22 (1992).
Wass, C.T., and Lanier, W.L., *Mayo Clinic Proceedings* 71:801-812 (1996).
Weir, C.J., *et al., BMJ* 314 (7090):1303-6 (1997).
Widmann, C*., et al., Mol. Pharmacol.* 45:1029-1035 (1994).
U.S. Patent Nos. 4,710,473; 4,923,967; 5,118,666; 5,188,666; 5,120,712; 5,284,656; 5,364,838; 5,545,618; 5,512,549; 5,523,549.
Foreign Patent Nos. WO 91/11457; WO 96/29342; WO 96/25487; WO 96/19197.

## Claims

1. The use of a compound selected from GLP-1, GLP-1 analogues, GLP-1 derivatives, and pharmaceutically acceptable salts thereof, at a dose effective to normalize blood glucose in the manufacture of a medicament to reduce mortality and morbidity after stroke.

2. The use of a compound according to claim 1, wherein the compound is intravenously administered.

3. The use of a compound according to claim 1, wherein the compound is administered subcutaneously.

4. The use of a compound according to claims 2 or 3, wherein the administration is continuous.

5. The use of a compound according to claim 4, wherein the rate of administration of the compound is between 0.25 and 6 pmol/kg/min.

6. The use of a compound according to claim 5, wherein the rate of administration of the compound is between 0.5 and 2.4 pmol/kg/min.

7. The use of a compound according to claim 6, wherein the rate of administration of the compound is between 0.5 and 1.2 pmol/kg/min.

8. The use of a compound according to claim 2, wherein the intravenous administration is intermittent.

9. The use of a compound according to claim 2, wherein the compound is administered intravenously and also administered by another parenteral route.

10. The use of a compound according to claim 9, wherein the other parenteral route is the subcutaneous route.

11. The use of a compound according to claim 1, wherein the compound administered is GLP(7-36)amide, or a pharmaceutically acceptable salt thereof.

12. The use of a compound that exerts insulinotropic activity by interacting with the same receptor, or receptors with which GLP-1, GLP-1 analogues and GLP-1 derivatives interact in exerting their insulinotropic activity in the manufacture of a medicament to reduce mortality and morbidity after stroke.

13. The use of a compound that enhances insulin sensitivity by interacting with the same receptor, or receptors with which GLP-1, GLP-1 analogues and GLP-1 derivatives interact to enhance insulin sensitivity in the manufacture of a medicament to reduce mortality and morbidity after stroke.
